# EUROPÄISCHE PATENTSCHRIFT

(11) **EP 3 497 075 B1**
(45) Veröffentlichungstag und Bekanntmachung des Hinweises auf die Patenterteilung: **05.08.2020**
(21) Anmeldenummer: 17746511.9
(22) Anmeldetag: 07.08.2017
(51) Int. Cl.: C07C 37/00, C07C 39/20

(54) **VERFAHREN ZUR HERSTELLUNG VON SUBSTITUIERTEN STYROL-DERIVATEN**
METHOD FOR THE PREPARATION OF SUBSTITUTED STYRENE DERIVATIVES
PROCÉDÉ DE FABRICATION DE DÉRIVÉS DE STYRENE SUBSTITUÉS

(30) Priorität: 12.08.2016 EP 16183948
(43) Veröffentlichungstag der Anmeldung: 19.06.2019
(73) Patentinhaber: Bayer CropScience Aktiengesellschaft, 40789 Monheim am Rhein (DE)
(72) Erfinder: GALLENKAMP, Daniel, 42113 Wuppertal (DE); FORD, Mark James, 65207 Wiesbaden-Breckenheim (DE)
(74) Vertreter: BIP Patents
(86) Internationale Anmeldenummer: PCT/EP2017/069926
(87) Internationale Veröffentlichungsnummer: WO 2018/029140

(56) Entgegenhaltungen:
- WO-A1-2015/189114
- F. DUBNIKOVA ET.AL.: "Isomerisation of dihydrobenzofuran and isodihydrobenzofuran. Quantum chemical and kinetics calculations", J. PHYS. CHEM. A, Bd. 106, 2002, Seiten 9278-9283, XP002766440,
- R. HUISGEN ET.AL.: "Neue Ringschlüsse über Arine", CHEM. BER., Bd. 93, 1. Januar 1960 (1960-01-01), Seiten 1496-1506, XP002766442, in der Anmeldung erwähnt

## Beschreibung

Die vorliegende Erfindung betrifft ein Verfahren zur Herstellung von substituierten Styrol-Derivaten.

Es ist bereits bekannt, dass substituierte Styrol-Derivate nützliche Zwischenstufen bei der Herstellung agrochemischer Wirkstoffe sind (siehe beispielsweise WO 2012/025557).

Es sind verschiedene Verfahren zur Herstellung solcher substituierter Styrol-Derivate in der Literatur beschrieben.

Ein mögliches Verfahren zur Herstellung von 3-Chlor-2-vinylphenol ist in WO 2015/189114 bzw. US5424460 beschrieben. Die Herstellung erfolgt durch Aromatisierung von 2,2,6-Trichlor-1-vinyl-7-oxabicyclo[4.1.0]heptan unter basischen Bedingungen. Nachteilig an diesem Verfahren ist die Verwendung eines organometallischen Reagenz für die Darstellung von 2,2,6-Trichlor-1-vinyl-7-oxabicyclo[4.1.0]heptan sowie die Tendenz zu Oligomer- bzw. Polymerbildung bei der Darstellung dieser Vorstufe.

Eine alternative und allgemeine Möglichkeit zur Darstellung von 2-Vinylphenolen besteht in der Ringöffnung von 2,3-Dihydrobenzofuranen unter basischen Bedingungen. Beispielsweise liefert die Behandlung von 2,3-Dihydrobenzofuran mit 2.3 Equivalenten LiNH₂ als Base in HMPA oder DMSO bei einer Temperatur von 20 bis 60 °C und einer Reaktionszeit von fünf Stunden 2-Vinylphenol mit einer Ausbeute von 95% (Doklady Akademii Nauk SSSR 1978, 239, 1357). Nachteilig bei diesem Verfahren ist die Verwendung von LiNH₂ als sehr starke Base sowie HMPA oder DMSO als Lösungsmittel, da diese Stoffe für den technischen Gebrauch ungeeignet sind. Die Behandlung von 2,3-Dihydrobenzofuran mit 1.5 Equivalenten LiNEt₂ als Base in Diethylether bei einer Temperatur von 35 °C und einer Reaktionszeit von 45 Stunden liefert 2-Vinylphenol mit einer Ausbeute von 25% (Chem. Ber. 1960, 93, 1496). Nachteilig an diesem Verfahren ist die Verwendung von LiNEt₂ als sehr starke Base sowie Diethylether als Lösungsmittel, da diese Stoffe für den technischen Gebrauch ungeeignet sind. Darüber hinaus wird durch das beschriebene Verfahren eine geringe Ausbeute von 25% von 2-Vinylphenol erzielt und sehr lange Reaktionszeit benötigt. Aus diesen Gründen ist das Verfahren für einen technischen Gebrauch ungeeignet.

Wegen der Bedeutung von substituierten Styrol-Derivaten als Baustein zur Synthese neuer agrochemischer Wirkstoffe besteht die Aufgabe darin, ein Verfahren zu finden, das großtechnisch und kostengünstig eingesetzt werden kann und die oben beschriebenen Nachteile umgeht. Auch ist es erstrebenswert, die speziellen Styrol-Derivate mit hoher Ausbeute und hoher Reinheit zu erhalten, so dass die Zielverbindung vorzugsweise keiner weiteren möglicherweise komplexen Aufreinigung unterzogen werden muss.

Diese Aufgabe wurde gelöst durch ein Verfahren zur Herstellung von substituierten Styrol-Derivaten der Formel **(I):** 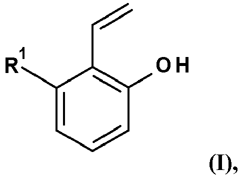 in der
R¹ für Cl **(Ia)**, Br **(Ib)** oder Methyl (**Ic**) steht,
dadurch gekennzeichnet dass ein Dihydrobenzofuran-Derivat der Formel (**II**) 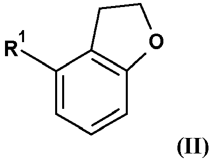 in der
R¹ für Cl, Br oder Methyl steht
durch Erhitzen in Gegenwart einer Alkoxid oder Hydroxid Base
zu Verbindungen der Formel (I) umgesetzt wird.

Bevorzugt ist ein erfindungsgemäßes Verfahren, bei dem die Restedefinitionen der Formeln **(I)** und (**II**) wie folgt sind:
- R¹: steht für Cl.

### Beschreibung des Prozesses

Die erfindungsgemäße Reaktion ist in Schema 1 dargestellt.

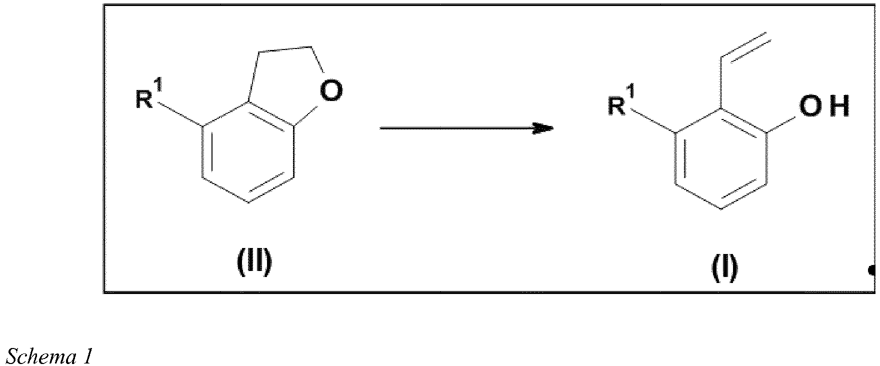

Die gewünschten Styrol-Derivate der allgemeinen Formel (I) werden mit dem erfindungsgemäßen Verfahren mit guten Ausbeuten und in hoher Reinheit erhalten.

Das erfindungsgemäße Verfahren hat gegenüber den vorab beschriebenen Verfahren den Vorteil, dass die Ausgangsstoffe im technischen Maßstab herstellbar sind und das Verfahren überraschenderweise durch Verwendung schwächerer Basen wie beispielsweise Kaliumhydroxid erfolgreich durchgeführt werden kann.

Als Lösungsmittel für das erfindungsgemäße Verfahren kommen grundsätzlich alle unter den Reaktionsbedingungen inerten organischen Lösungsmittel oder Lösungsmittelgemische in Frage, unter anderem: Ether (wie z.B.: 1,2-Dimethoxyethan (DME), Diglyme, Tetrahydrofuran (THF), 2-Methyl-THF, Anisol und 1,4-Dioxan); Amid Lösungsmittel (wie z.B.: DMF, *N*,*N*-Dimethylacetamid (DMAc)) und dipolar aprotische Lösungsmittel (wie z.B.: DMSO). Bevorzugt wird THF, 2-Methyl-THF, Dioxan, DME, Anisol, Diglyme, DMF oder DMAc oder Gemische aus DMAc mit Ethern oder Gemische aus DMAc mit aromatischen Lösungsmitteln (wie z.B.:Toluol, Xylol, Chlorbenzol oder 1,2-Dichlorbenzol) verwendet. Besonders bevorzugt verwendet man DMAc, DMF oder Diglyme oder Gemische aus DMAc oder DMF mit THF, 2-Methyl-THF, Dioxan, DME, Toluol, Xylol, Chlorbenzol oder 1,2-Dichlorbenzol.

Als Alkoxid Base kommen für das erfindungsgemäße Verfahren beispielsweise die folgenden in Frage: Kalium-*tert*-butylat, Natrium-*tert*-butylat, Natriummethylat, Natriumethylat oder Kaliummethylat. Als Hydroxid Basen kommen für das erfindungsgemäße Verfahren beispielsweise die folgende in Frage: Kaliumhydroxid. Bevorzugt wird Kaliumhydroxid, Kalium-*tert*-butylat, Natrium-*tert*-butylat oder Natriummethylat verwendet. Besonders bevorzugt wird Kaliumhydroxid oder Kalium-*tert*-butylat verwendet.

Besonders bevorzugt sind folgende Kombinationen aus den oben beschriebenen Gruppen der Lösungsmittel und Basen:
a) Diglyme in Kombination mit Kaliumhydroxid
b) DMAc in Kombination mit Kaliumhydroxid
c) DMF in Kombination mit Kaliumhydroxid
d) Lösungsmittelgemisch von DMAc mit Toluol, Xylol, Chlorbenzol, 1,2-Dichlorbenzol, Dioxan, DME, 2-Methyl-THF oder THF in Kombination mit Kaliumhydroxid
e) Lösungsmittelgemisch von DMF mit Toluol, Xylol, Chlorbenzol, 1,2-Dichlorbenzol, Dioxan, DME, 2-Methyl-THF oder THF in Kombination mit Kaliumhydroxid
f) DMAc, DMF, THF, 2-Methyl-THF, Dioxan, DME, Anisol oder Diglyme oder Gemische dieser Lösungsmittel in Kombination mit Kalium-*tert*-butylat.

Ganz besonders bevorzugt für das erfindungsgemäße Verfahren sind die folgende Kombination aus Lösungsmittel und Base: DMAc oder Diglyme und Kaliumhydroxid.

Die Temperatur des erfindungsgemäßen Verfahrens kann in weiten Grenzen variiert werden. Üblicherweise arbeitet man bei einer Temperatur von 20 °C bis 120 °C. Bevorzugt wird die Umsetzung bei einer Temperatur im Bereich von 80 °C bis 120 °C durchgeführt. Besonders bevorzugt wird die Umsetzung bei einer Temperatur im Bereich von 100 °C bis 120 °C durchgeführt.

Üblicherweise wird das erfindungsgemäße Verfahren bei Normaldruck durchgeführt. Es ist auch möglich die Reaktion im Vakuum oder bei erhöhtem Druck (Überdruck) durchzuführen.

Die molaren Verhältnisse der Verbindung der Formel (**II**) zu Basen der oben beschriebenen Gruppe kann in weiten Grenzen variiert werden. Üblicherweise arbeitet man mit einem molaren Verhältnis von 1:1 bis 1:5. Für organische Basen wie beispielsweise Kalium-tert-butylat wird ein molares Verhältnis von 1:1 bis 1:1,5 bevorzugt. Besonders bevorzugt wird ein molares Verhältnis von 1:1,1. Für anorganische Basen wie beispielsweise Kaliumhydroxid wird ein molares Verhältnis von 1:2 bis 1:4 bevorzugt. Besonders bevorzugt wird ein molares Verhältnis von 1:3.

Die Reaktionsdauer der Reaktion ist kurz und liegt im Bereich von etwa 0,5 bis etwa 5 Stunden. Eine längere Reaktionsdauer ist möglich, jedoch wirtschaftlich nicht sinnvoll.

Die Verbindungen der Formel (**I**) werden nicht isoliert. Die Verbindungen der Formel (**I**) werden entweder direkt (bei Verwendung von Kalium-*tert*-butylat als Base) oder nach einer Zwischenaufarbeitung (bei Verwendung von Kaliumhydroxid als Base) weiter umgesetzt zu Verbindungen der Formel (**III**) (Schema 2). Die Umsetzung zu Verbindungen der Formel (**III**) erfolgt wie in WO 2015/189114 beschrieben.

Bevorzugt wird die folgende Reaktionssequenz zur Herstellung der Verbindung der Formel (**III**) durchgeführt:

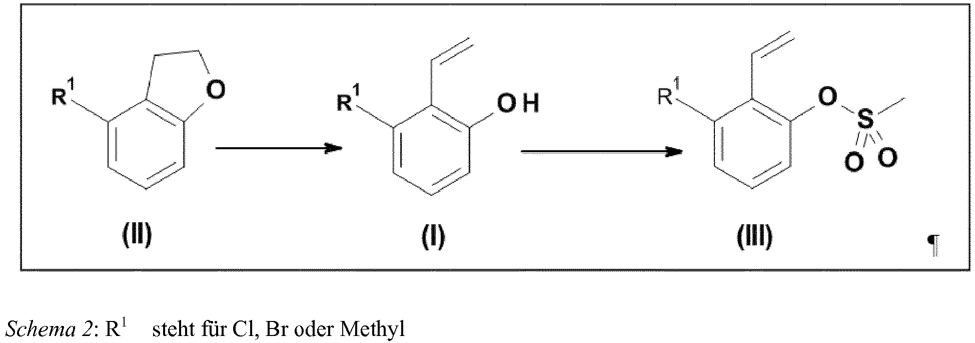

### Beispiele

Die vorliegende Erfindung wird anhand der nachfolgenden Beispiele näher erläutert, ohne die Erfindung dabei auf diese einzuschränken.

### 3-Chlor-2-vinylphenol (Ia)

Eine Lösung von 4-Chlor-2,3-dihydro-1-benzofuran (**IIa**) (19.0 g, 114.3 mmol) in *N*,*N*-Dimethylacetamid (75 ml) wurde bei 20 °C mit Kaliumhydroxid Pulver (85%, 22.6 g, 342.9 mmol) versetzt, auf 120 °C erhitzt und 1 h bei dieser Temperatur gerührt. Das Reaktionsgemisch wurde auf ca. 10 - 15 °C abgekühlt, mit Wasser verdünnt (150 ml) und durch langsame Zugabe von 37% Salzsäure (40 ml) auf einen pH-Wert von 1 gebracht. Die wässrige Phase wurde zweimal mit Toluol extrahiert (je 100 ml), die vereinigten organischen Phasen einmal mit 10% Salzsäure (30 ml) gewaschen und im Vakuum auf ein Restvolumen von ca. 100 ml eingeengt. Die so erhaltene Lösung wird direkt im nächsten Schritt eingesetzt. Analytische Daten von 3-Chlor-2-vinylphenol sind wie folgt: ¹H-NMR (CDCl₃, 400 MHz) δ (ppm) = 7.08 (dd, *J =* 8.0, 8.0 Hz, 1H), 6.96 (d, *J* = 8.0 Hz, 1H), 6.84 (d, *J* = 8.0 Hz, 1H), 6.79 (dd, *J* = 12.0 Hz, 12.0 Hz, 1H), 5.74 (d, *J =* 12.0 Hz, 1H), 5.73 (s, 1H), 5.68 (d, *J =* 12.0 Hz, 1H).

### 3-Brom-2-vinylphenol (Ib)

¹H-NMR (DMSO-d6, 400 MHz) δ (ppm) = 10.20 (s, 1H), 7.08 (dd, J = 8.0, 1.3 Hz, 1H), 6.99 (t, J = 8.0 Hz, 1H), 6.89 (dd, J = 8.0, 1.0 Hz, 1H), 6.77 (dd, J = 17.8, 12.1 Hz, 1H), 6.08 (dd, J = 17.8, 2.5 Hz, 1H), 5.51 (dd, J = 12.1, 2.5 Hz, 1H).

### 3-Methyl-2-vinylphenol (Ic)

¹H-NMR (DMSO-d6, 400 MHz) δ (ppm) = 9.45 (s, 1H), 6.93 (t, J = 7.8 Hz, 1H), 6.73 (dd, J = 17.9, 11.8 Hz, 1H), 6.69 (d, J = 7.8, 1.0 Hz, 1H), 6.63 (d, J = 7.8 Hz, 1H), 5.76 (dd, J = 17.9, 2.5 Hz, 1H), 5.41 (dd, J = 11.9, 2.5 Hz, 1H), 2.27 (s, 3H).

### 3-Chlor-2-vinylphenyl-methansulfonat (IIIa)

Die im obigen Schritt erhaltene Toluol Lösung von **Ia** wurde auf 0 - 5 °C abgekühlt und mit Triethylamin versetzt (17.5 ml, 125.7 mmol). Eine 50% Lösung von Methansulfonylchlorid (9.7 ml, 125.7 mmol) in Toluol wurde über einen Zeitraum von 1h und einer Temperatur von 0 - 5 °C zudosiert und nach beendeter Zugabe 30 min bei 20 °C gerührt. Anschließend wurde das Reaktionsgemisch durch die langsame Zugabe von 10% Salzsäure (50 ml) bei 10 - 15 °C auf einen pH-Wert von 1 gebracht und die wässrige Phase zweimal mit einer Mischung aus Toluol / *tert*-Butyl-methylether 4:1 extrahiert (je 50 ml). Die vereinigten organischen Phasen wurden im Vakuum eingeengt und der Rückstand aus Ethanol (15 ml) umkristallisiert (23.7 g, 89% der Theorie). ¹H-NMR (CDCl₃, 400 MHz) δ (ppm) = 7.36 (dd, *J =* 8.0, 1.2 Hz, 1H), 7.34 (dd, *J* = 8.0, 1.2 Hz, 1H), 7.23 (t, *J* = 8.0 Hz, 1H), 6.76 (dd, *J* = 18.0 Hz, 11.7 Hz, 1H), 5.91 (dd, *J* = 18.0, 1.6 Hz, 1H), 5.73 (dd, *J* = 11.8, 1.4 Hz, 1H), 3.11 (s, 3H).

### 3-Brom-2-vinylphenyl-methansulfonat (IIIb)

¹H-NMR (CDCl₃, 400 MHz) δ (ppm) = 7.56 (dd, *J* = 8.1, 1.1 Hz, 1H), 7.38 (dd, *J =* 8.1, 1.1 Hz, 1H), 7.16 (t, *J =* 8.1 Hz, 1H), 6.72 (dd, *J* = 18.0 Hz, 11.8 Hz, 1H), 5.84 (dd, *J* = 18.0, 1.4 Hz, 1H), 5.71 (dd, *J =* 11.6, 1.4 Hz, 1H), 3.11 (s, 3H).

### 3-Methyl-2-vinylphenyl-methansulfonat (IIIc)

¹H-NMR (CDCl₃, 400 MHz) δ (ppm) = 7.23 (dd, *J =* 7.8, 1.8 Hz, 1H), 7.18 (t, *J =* 7.8 Hz, 1H), 7.14 (dd, *J* = 7.5, 1.4 Hz, 1H), 6.71 (dd, *J* = 17.8 Hz, 11.8 Hz, 1H), 5.65 (dd, *J =* 11.8, 1.6 Hz, 1H), 5.63 (dd, *J* = 17.8, 1.6 Hz, 1H), 3.09 (s, 3H), 2.37 (s, 3H).

## Patentansprüche

1. Verfahren zur Herstellung von substituierten Styrol-Derivaten der Formel (I): 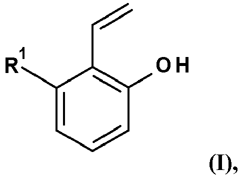 in der
R¹ für Cl (**Ia**), Br **(Ib)** oder Methyl (**Ic**) steht,
**dadurch gekennzeichnet dass** ein Dihydrobenzofuran-Derivat der Formel (**II**) 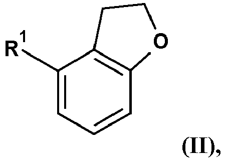 in der
R¹ für Cl, Br oder Methyl steht,
durch Erhitzen in Gegenwart einer Alkoxid oder Hydroxid Base
zu Verbindungen der Formel **(I)** umgesetzt wird.

2. Verfahren nach Anspruch 1, **dadurch gekennzeichnet, dass**
die Restedefinitionen der Formeln **(I)** und (**II**) wie folgt sind:
R¹ steht für Cl.

3. Verfahren nach einem der Ansprüche 1 oder 2, **dadurch gekennzeichnet, dass** als Base Kalium-tert-butylat, Natrium-tert-butylat, Natriummethylat, Natriumethylat, Kaliummethylat oder Kaliumhydroxid verwendet wird.

4. Verfahren nach einem der Ansprüche 1 bis 3 , **dadurch gekennzeichnet, dass** als Lösungsmittel ein Amid oder Ether Lösungsmittel oder Gemische von Amid Lösungsmitteln mit Ethern oder aromatischen Lösungsmitteln verwendet werden.

5. Verfahren nach einem der Ansprüche 1 bis 3, **dadurch gekennzeichnet, dass** als Lösungsmittel DMAc, DMF oder Diglyme oder Gemische aus DMAc oder DMF mit THF, 2-Methyl-THF, Dioxan, DME, Toluol, Xylol, Chlorbenzol oder 1,2-Dichlorbenzol verwendet werden.

6. Verfahren nach einem der Ansprüche 1 bis 3, **dadurch gekennzeichnet, dass** eine der folgenden Kombinationen aus Lösungsmittel und Base für die Reaktion verwendet wird:
a) Diglyme in Kombination mit Kaliumhydroxid
b) DMAc in Kombination mit Kaliumhydroxid
c) DMF in Kombination mit Kaliumhydroxid
d) Lösungsmittelgemisch von DMAc mit Toluol, Xylol, Chlorbenzol, 1,2-Dichlorbenzol, Dioxan, DME, 2-Methyl-THF oder THF in Kombination mit Kaliumhydroxid
e) Lösungsmittelgemisch von DMF mit Toluol, Xylol, Chlorbenzol, 1,2-Dichlorbenzol, Dioxan, DME, 2-Methyl-THF oder THF in Kombination mit Kaliumhydroxid
f) DMAc, DMF, THF, 2-Methyl-THF, Dioxan, DME, Anisol oder Diglyme oder Gemische dieser Lösungsmittel in Kombination mit Kalium-*tert*-butylat

7. Verfahren nach einem der Ansprüche 1 bis 5, **dadurch gekennzeichnet, dass** als anorganische Base Kaliumhydroxid, als organische Base Kalium-tert-butylat verwendet wird.

## Claims

1. Method for preparing substituted styrene derivatives of the formula **(I):** 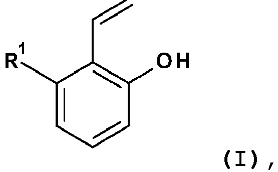 where
R¹ is Cl (**Ia**), Br (**Ib**) or methyl (Ic), **characterized in that** a dihydrobenzofuran derivative of the formula **(II)** 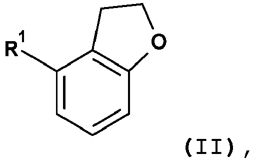 where
R¹ is Cl, Br or methyl,
is reacted by heating in the presence of an alkoxide or hydroxide base
to give compounds of the formula **(I).**

2. Method according to Claim 1, **characterized in that** the radical definitions of the formulae **(I)** and **(II)** are as follows:
R¹ is Cl.

3. Method according to either of Claims 1 and 2, **characterized in that** the base used is potassium *tert*-butoxide, sodium *tert*-butoxide, sodium methoxide, sodium ethoxide, potassium methoxide or potassium hydroxide.

4. Method according to any of Claims 1 to 3, **characterized in that** an amide or ether solvent or mixtures of amide solvents with ethers or aromatic solvents are used as solvent.

5. Method according to any of Claims 1 to 3, **characterized in that** the solvents used are DMAc, DMF or diglyme or mixtures of DMAc or DMF with THF, 2-methyl-THF, dioxane, DME, toluene, xylene, chlorobenzene or 1,2-dichlorobenzene.

6. Method according to any of Claims 1 to 3, **characterized in that** one of the following combinations of solvent and base is used for the reaction:
a) diglyme in combination with potassium hydroxide
b) DMAc in combination with potassium hydroxide
c) DMF in combination with potassium hydroxide
d) solvent mixture of DMAc with toluene, xylene, chlorobenzene, 1,2-dichlorobenzene, dioxane, DME, 2-methyl-THF or THF in combination with potassium hydroxide
e) solvent mixture of DMF with toluene, xylene, chlorobenzene, 1,2-dichlorobenzene, dioxane, DME, 2-methyl-THF or THF in combination with potassium hydroxide
f) DMAc, DMF, THF, 2-methyl-THF, dioxane, DME, anisole or diglyme or mixtures of these solvents in combination with potassium tert-butoxide.

7. Method according to any of Claims 1 to 5, **characterized in that** potassium hydroxide is used as inorganic base and potassium tert-butoxide is used as organic base.

## Revendications

1. Procédé pour la préparation de dérivés de styrène substitués de formule (I) : 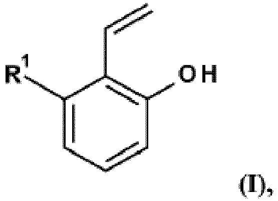 dans laquelle
R¹ représente Cl (Ia), Br (Ib) ou méthyle (Ic), **caractérisé en ce qu'**un dérivé de dihydrobenzofuranne de formule (II) 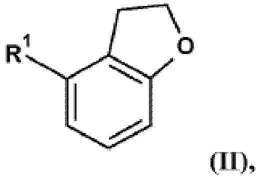 dans laquelle
R¹ représente Cl, Br ou méthyle,
est transformé par chauffage en présence d'une base de type alcoxyde ou hydroxyde en des composés de formule (I).

2. Procédé selon la revendication 1, **caractérisé en ce que** les définitions de radicaux des formules (I) et (II) sont comme suit :
R¹ représente Cl.

3. Procédé selon l'une quelconque des revendications 1 et 2, **caractérisé en ce que** du *tert*-butylate de potassium, du *tert*-butylate de sodium, du méthylate de sodium, de l'éthylate de sodium, du méthylate de potassium ou de l'hydroxyde de potassium est utilisé en tant que base.

4. Procédé selon l'une quelconque des revendications 1 à 3, caractérisé en que qu'un solvant de type amide ou éther ou des mélanges de solvants de type amide avec des éthers ou avec des solvants aromatiques sont utilisés en tant que solvant.

5. Procédé selon l'une quelconque des revendications 1 à 3, **caractérisé en ce que** du DMAc, du DMF ou du diglyme ou des mélanges de DMAc ou de DMF avec du THF, du 2-méthyl-THF, du dioxanne, du DME, du toluène, du xylène, du chlorobenzène ou du 1,2-dichlorobenzène sont utilisés en tant que solvant.

6. Procédé selon l'une quelconque des revendications 1 à 3, **caractérisé en ce qu'**une des combinaisons suivantes de solvant et de base est utilisée pour la réaction :
a) diglyme en combinaison avec de l'hydroxyde de potassium
b) DMAc en combinaison avec de l'hydroxyde de potassium
c) DMF en combinaison avec de l'hydroxyde de potassium
d) mélange de solvant de DMAc avec du toluène, du xylène, du chlorobenzène, du 1,2-dichlorobenzène, du dioxanne, du DME, du 2-méthyl-THF ou du THF en combinaison avec de l'hydroxyde de potassium
e) mélange de solvant de DMF avec du toluène, du xylène, du chlorobenzène, du 1,2-dichlorobenzène, du dioxanne, du DME, du 2-méthyl-THF ou du THF en combinaison avec de l'hydroxyde de potassium
f) DMAc, DMF, THF, 2-méthyl-THF, dioxanne, DME, anisole ou diglyme ou des mélanges de ces solvants en combinaison avec du *tert*-butylate de potassium.

7. Procédé selon l'une quelconque des revendications 1 à 5, **caractérisé en ce que** de l'hydroxyde de potassium est utilisé en tant que base inorganique, du *tert*-butylate de potassium est utilisé en tant que base organique.
